# EUROPEAN PATENT APPLICATION

(11) **EP 3 954 226 A1**
(43) Date of publication of application: **16.02.2022**
(21) Application number: 21190856.1
(22) Date of filing: 11.08.2021
(51) Int. Cl.: A23L 33/16, A61K 8/19, A61K 9/14, A61K 9/20, A61K 33/00, A61K 33/06, A61K 33/24, A61K 31/00

(54) **ATOMIZED COLLOIDAL MINERAL SOLUTION AND COMPOSITIONS COMPRISING THE SAME**

(30) Priority: 13.08.2020 IT 202000020098; 09.11.2020 IT 202000026630
(71) Applicant: Cabassi & Giuriati S.p.A., 35127 Padova (PD) (IT)
(72) Inventor: GIURIATI, Daniela, PADOVA (IT); COATTO, Massimo, PADOVA (IT)
(74) Representative: Rigamonti, Dorotea

(57) **Abstract**

The present invention relates to a formulation which comprises an atomized vegetable colloidal solution loaded on cyclodextrins, wherein said colloidal solution comprises calcium, chlorine, phosphorus, magnesium, potassium, sodium and, optionally, iron, manganese, zinc, copper and / or selenium. Compositions comprising said formulation and their use form a further object of the present invention.

## Description

### Background

Cyclodextrins are natural products consisting of glucopyranose units linked in a cyclic form to form toroid structures. In addition to some minor cyclic oligosaccharides, cyclodextrins comprise 3 main natural products, defined α-cyclodextrins, formed by six glucose subunits, β-cydodextrins, formed by seven subunits, and γ-cydodextrins, which have eight subunits. The secondary hydroxyl groups of glucopyranose (C2 and C3) are located at the level of the margin that delineates the larger opening of the toroid while the primary hydroxyls (C6) are located around the smaller edge. The cyclic structure is shaped like a toroid, whose cavity (hydrophobic) is made up of hydrogen atoms and the hydrogen bonds between the glycosidic H and O. The side hosting the secondary hydroxyl groups has a larger diameter than that with the primary hydroxyls, as these are allowed free rotation which causes the diameter to be reduced.

In aqueous solution, the cavity of the cyclodextrins is occupied by water molecules: since it is an interaction between a polar and an apolar molecule, water can be easily replaced by other molecules. What determines the formation of the complex is the replacement of the water molecules with other molecules of lower polarity. The cavity of cyclodextrins can host various molecules depending on their chemical-physical characteristics. Due to these properties, cyclodextrins are used in pharmaceutical technology to increase the water solubility of molecules through the formation of inclusion complexes, as well as for their protective capability of unstable molecules mainly in aqueous environment, where cyclodextrins subtract the molecules included in the degradation equilibria. Cyclodextrins are widely used in oral formulations, also due to their proven absence of toxicity.

Planta Mineral Toddy (# 17601 Nutriva, Cabassi & Giuriati SpA IT) is a vegetable colloidal solution rich in organic minerals.

There is a strong need for new formulations capable of delivering the trace elements that characterize this solution in a stable manner by increasing their bioavailability.

### Description

The object of the present invention is a formulation which comprises an atomized vegetable colloidal solution loaded on cyclodextrins.

For the purpose of the present invention, by vegetable colloidal solution is meant an aqueous solution extracted from a mine of fossil vegetable material. In one embodiment, said colloidal solution comprises:

| | |
|---|---|
| Calcium | > 100 mg / l |
| Chlorine | about 8 mg / l |
| Phosphorus | about 12 mg / l |
| Magnesium | > 100 mg / l |
| Potassium | > 100 mg / l |
| Sodium | > 100 mg / l |

In one embodiment, said colloidal solution also comprises at least one of iron, manganese, zinc. Optionally, it also comprises at least one of copper, selenium. In one embodiment, said colloidal solution comprises calcium, chlorine, phosphorus, magnesium, potassium, sodium, iron, manganese, zinc and at least one of copper and selenium.

In one embodiment, said solution comprises:

| | |
|---|---|
| Calcium | > 100 mg / l |
| Chlorine | about 8 mg / l |
| Phosphorus | about 12 mg / l |
| Magnesium | > 100 mg / l |
| Potassium | > 100 mg / l |
| Sodium | > 100 mg / l |
| Total iron | 250 mg / l |
| Manganese | about 9 mg / l |
| Zinc | > 50 mg / l |
| Copper | between 0 and 0.400 mg / l |
| Selenium | between 0 and 0.400 mg / l |

In one embodiment, said solution comprises:

| | |
|---|---|
| Calcium | between 100 and 400 mg / l |
| Chlorine | about 8 mg / l |
| Phosphorus | about 12 mg / l |
| Magnesium | > 100 mg / l |
| Potassium | > 100 mg / l |
| Sodium | > 100 mg / l |
| Total iron | 250 mg / l |
| Manganese | about 9 mg / l |
| Zinc | > 50 mg / l |
| Copper | between 0 and 0.400 mg / l |
| Selenium | between 0 and 0.400 mg / l |
| In one embodiment, said solution comprises | |
| Calcium | about 160 mg / l |
| Chlorine | about 8 mg / l |
| Phosphorus | about 12 mg / l |
| Magnesium | between 100 and 600 mg / l |
| Potassium | between 100 and 400 mg / l |
| Sodium | between 100 and 400 mg / l |
| Total iron | between 250 and 600 mg / l |
| Manganese | about 9 mg / l |
| Zinc | > 50 mg / l |
| Copper | between 0 and 0.400 mg / l |
| Selenium | between 0 and 0.400 mg / l |

In a preferred form, said colloidal solution is Plant Mineral Toddy (# 17601 Nutriva, Cabassi & Giuriati SpA IT).

Said colloidal solution is atomized on cyclodextrins.

Said cyclodextrins are selected from alpha-cyclodextrin, beta-cyclodextrin, gamma-cyclodextrin, hydroxypropyl-beta-cyclodextrin, hydroxypropyl-gamma-cyclodextrin, other derivatives of cyclodextrin and their derivatives or mixtures.

In one embodiment, said cyclodextrin is alpha-cyclodextrin.

Said embodiment is particularly advantageous when said formulation is for food use.

In one embodiment, said cyclodextrin is a beta-cyclodextrin.

Said embodiment is particularly advantageous when said formulation is for cosmetic use.

For the purpose of the present description, cyclodextrins loaded with the powder obtained from the atomization of an aqueous colloidal solution which comprises: Calcium> 100 mg / l, Chlorine about 8 mg / l, Phosphorus about 12 mg / l, Magnesium> 100 mg / l, Potassium> 100 mg / l, Sodium> 100 mg / I, are called C2P.

Said atomization process takes place according to methods known to the expert in the field.

Said process leads to obtaining a powder loaded with the minerals of which said colloidal solution is rich.

Advantageously, a powder formulation is made available which comprises and keeps unaltered the components of the original colloidal solution.

Said powder formulation is advantageously used in food supplements, or in cosmetic products, by way of example it is added to creams, gels, lotions. Advantageously, said formulation is diluted in powder preparations such as, by way of example, talc, starch.

In an embodiment, said formulation which comprises cyclodextrins loaded with said atomized colloidal solution is previously loaded onto a powder excipient selected from maltodextrin, starch, polydextrose. This dilution process leads to obtaining volumes of powder loaded with said atomized colloidal solution useful for the preparation of food supplements and / or cosmetic products.

Advantageously, the formulation according to the present invention is used in compositions that are food supplements, in which it guarantees a stable content over time and a controlled release after the assumption of the components of the starting colloidal solution.

Said formulation is advantageously used in compositions for the preparation of beverages which are replenishers of mineral salts.

In cosmetics, an advantageous use consists in diluting the formulation according to the present invention in distilled water, obtaining a water with a controlled and stable content which is tonic for the skin and which is conveniently used alone, or in the formulation of gels, creams, lotions.

A further object of the present invention is a composition which comprises:
a) C2P: cyclodextrins loaded with the powder obtained from the atomization of an aqueous colloidal solution which comprises:

| | |
|---|---|
| Calcium | > 100 mg / l |
| Chlorine | about 8 mg / l |
| Phosphorus | about 12 mg / l |
| Magnesium | > 100 mg / l |
| Potassium | > 100 mg / l |
| Sodium | > 100 mg / l |

b) one or more additional actives;
c) pharmaceutically acceptable excipients.

In one embodiment, said composition is in the form of a powder, tablet, aqueous solution, oily solution, or capsule.

In one embodiment, said composition is for oral administration.

In one embodiment, said composition is for topical use.

Alternative embodiments of the composition according to the present invention are described below. Unless otherwise indicated, the % are to be understood w / V.

In one embodiment, said composition is in the pharmaceutical form of tablets and comprises: C2P in an amount between 0.1 and 40% (w / V), one or more further active 0.1-40% (w / V), bulking agents 1-50% (w / V), binders 5-40% (w / V), disintegrating agents 1-40% (w / V), sweeteners 0-20% (w / V), anti-aggregating agents 1- 5% (w / V), lubricants 1-5% (w / V), filming agents 0-5% (w / V), dyes 0-1% (w / V), flavours 0-1% (w / V).

In one embodiment, said composition is in the form of a powder and comprises C2P in an amount of between 0.1 and 40% (w / V), one or more further active 0.1-40%, bulking agents 1-50 %, 0-75% sweeteners, 1-5% antiplatelet agents, 5-10% buffering agents, 0-1% dyes, 0-10% flavours.

In one embodiment, said composition is an aqueous solution and comprises: C2P in an amount between 0.1 and 40% (w / V), one or more further active 0.1-10%, water 1-95%, bulking agents 1-50%, sweeteners 0-60%, buffering agents 5-10%, dyes 0-1%, flavours 0-10%, preservatives 0.05-0.2%, acidifiers 0.1-5% , thickeners 0.1-5%.

In one embodiment, said composition is an oily solution and comprises: C2P in an amount between 0.1 and 40% (w / V), one or more further active 0-10%, oils 1-95%, dyes 0-1%, 0-10% aromas, 0.1-5% antioxidants, 0.1-5% thickeners.

In one embodiment, said composition is in the form of capsules and comprises: C2P, optionally further actives, optionally pharmaceutically acceptable excipients.

The following examples have the sole purpose of better illustrating the invention, they are not intended to be in any way limitative of the same, the scope of which is defined by the claims.

### Examples

### Example 1 - Swallowable Tablet

1 kg of alpha cyclodextrin and 33 kg of colloidal solution which is Planta Mineral Toddy (# 17601 Nutriva, Cabassi & Giuriati SpA IT) are made available.

Said colloidal solution is subjected to an atomization process which leads to obtaining about 15 g of powder from the solutes contained therein. This powder is loaded onto 1 kg of alpha-cyclodextrin, leading to the obtainment of the formulation called C2P. C2P is advantageously inserted in a composition which is then formulated into tablet forms.

In addition to C2P, these tablets also comprise anhydrous magnesium tricitrate, in addition to the excipients according to Table 1.

**Table 1**

| **Ingredient** | **function** | **mg** | **%** |
|---|---|---|---|
| microcrystalline cellulose | | **754,000** | 58,00 |
| C2P | active | **200,000** | 15,39 |
| anhydrous magnesium tricitrate | active | **300,00** | 23,05 |
| magnesium stearate | | **34,500** | 2,66 |
| silicon dioxide | antiaggregrant | **11,500** | 0,88 |

The tablets are obtained with punches of the company Lurga (P) through compression in a Ronchi PA automatic rotary tablet press with 20 punches and have the specifications according to Table 2.

**Table 2**

| **Parameter** | **VALUE** |
|---|---|
| Hardness | >70 N |
| Friability | < 1 % |
| Thickness | 8 +/- 1 mm |
| Average weight | 1300 +/- 5% mg |

A GLOBOPHARMA S.A.S. durometer was used to measure diameter, thickness and hardness.

An ERWEKA TA phriabilometer was used to measure the friability. This measurement is the result of an average of three tests, each performed on 10 tablets, for each sample. Each test consists of 100 rotations equal to an estimated time of 4 minutes.

A RADWAG technical scale, model PS1000.R2, was used to measure the weight.

The reference for the methods is the ISO 9001: 2015 quality management system according to Table 3.

**Table 3**

| **Parameter** | **Method** |
|---|---|
| * hardness (N) | IO 10.41 Determination of the hardness and diameter of the tablets _Rev 02 |
| ** thickness (mm) | IO 10.41 Determination of the hardness and diameter of the tablets _Rev 02 |
| ***friability (%) | IO 10.22 Determination of the friability of the tablets _rev.01 |
| ****diameter (mm) | IO 10.41 Determination of the hardness and diameter of the tablets _Rev 02 |
| *****weight (mg) | IO 10.24 Determination of the average weight of the tablets _rev.01 |

In one embodiment, said tablets are melatonin tablets, characterized by the composition shown in Table 4.

**Table 4**

| **Ingredient** | **Amount (mg)** | **%** |
|---|---|---|
| Melatonin | 1 | 0,33 |
| Silicon dioxide | 1 | 0,33 |
| Magnesium stearate | 1 | 0,33 |
| Vitamin b6 HCl (pyridoxine HCI) | 2 | 0,66 |
| L-tryptophan | 20 | 6,66 |
| C2P | 100 | 33,33 |
| Microcrystalline cellulose | 175 | 58,33 |
| **Total** | **300 mg** | **100%** |

### Example 2 - Powder for oral solution

Cyclodextrins loaded as in example 1 are formulated according to Table 5.

**Table 5**

| **Ingredient** | **function** | **mg** | % |
|---|---|---|---|
| erythritol | sweetener | **2060.15** | 51.50 |
| C2P | active | **1000.00** | 25.00 |
| *Coleus Forskohlii* e.s. tit. 40% forskoline | active | **300.000** | 7.50 |
| I-carnitine base plv | active | **200.000** | 5.00 |
| mate' e.s. tit. 8% xantine | active | **200.000** | 5.00 |
| zinc gluconate | active | **113.000** | 2.83 |
| natural mandarin flavoring | flavour | **60.000** | 1.50 |
| Silicon dioxide | antiaggregant | **40.000** | 1.00 |
| anhydrous citric acid fg 51n | acidifier | **20.000** | 0.50 |
| stevia 98% | sweetener | **5.000** | 0.125 |
| chromium picolinate plv | active | **1.670** | 0.04 |
| sodium selenite plv | active | **0.180** | 0.005 |

The product is packaged in stick packs, obtained with the Universalpack pack 00B12 machine in polylaminate film of 85 micrometres thickness in PET / Alu / PE [12/8/65].

The tightness of the stick packs is assessed by immersing 4 stick packs in an aqueous solution of methylene blue contained in the special vacuum tool and bringing the pressure to 0.4 in Hg. The stick packs are left under vacuum for 3 minutes. The pressure is then brought back to atmospheric pressure and left to soak for another 3 minutes. Finally they are extracted, dried and opened to evaluate any infiltrations highlighted by the colour that methylene blue gives to the powder.

Advantageously, C2P guarantees the stable presence in said composition of the elements characterizing the starting colloidal solution, ensuring a controlled release after intake.

### Example 3 - chewable tablet

Cyclodextrins loaded as per example 1 are formulated according to Table 6, in the form of chewable tablets which include melatonin as active

**Table 6**

| **Ingredient** | **Amount (mg)** | % |
|---|---|---|
| Mannitol | **73** | **27.04** |
| Maltodextrin | **55** | **20.37** |
| Sucrosomal Zinc Ultrazin 40-50% | **25** | **9.25** |
| C2P | **20** | **7.40** |
| Sucrosomal Selenium Ultrasel 0.3% | **20** | **7.40** |
| Polydextrose | **16** | **5.92** |
| Honey powder | **15** | **5.55** |
| Microcrystalline cellulose | **13** | **4.81** |
| Chamomile spray extract | **10** | **3.70** |
| Silicon dioxide | **10** | **3.70** |
| Magnesium stearate | **10** | **3.70** |
| Safflower extract | **2** | **0.74** |
| Melatonin | **1** | **0.37** |
| Sucralose | **0.03** | **0.01** |
| **Total** | **270 mg** | **100%** |

### Example 4 - aqueous solution

Cyclodextrins loaded as per example 1 are formulated according to Table 7, in the form of an aqueous solution comprising Vitamin B12 as an active.

**Table 7**

| **Ingredient** | **Amount (mg)** | **%** |
|---|---|---|
| water | 26790 | 89.30 |
| C2P | 1500 | 5.00 |
| Vitamin B12 (Cyanocobalamin) | 1500 | 5.00 |
| Vitamin C (L-Ascorbic Acid) | 100 | 0.33 |
| Citric acid | 50 | 0.16 |
| Sodium benzoate | 30 | 0.10 |
| Potassium sorbate | 30 | 0.1 |
| **Total** | **30000 mg** | **100%** |

### Example 5 - oily solution

Cyclodextrins loaded as per example 1 are formulated according to Table 8, in the form of an oily solution comprising vitamin D3 as active.

**Table 8**

| **Ingredient** | **Amount (mg)** | % |
|---|---|---|
| Coconut oil (Di and triglycerides) | 25500mg | 85% |
| C2P | 3000mg | 10% |
| Cholecalciferol (Vitamin D3) | 25mcg-1000UI | 0,00008 |
| Vitamin E (D-Alpha-Tocopherol) | 1500mg | 5% |
| **Total** | **30000mg** | **100%** |

Oily solutions are conveniently formulated also in softgel capsules.

### Example 6 - capsules

Cyclodextrins loaded as per example 1 are formulated according to Table 9, in capsules which include mushroom powder as active ingredient.

**Table 9**

| **Ingredient** | **%** |
|---|---|
| mushroom powder | 83,2% |
| C2P | 0,9% |
| **Total** | **100%** |

## Claims

1. Formulation comprising cyclodextrins loaded with the powder obtained from the atomization of an aqueous colloidal solution which comprises:
| | |
|---|---|
| Calcium | > 100mg / l |
| Chlorine | about 8 mg / l |
| Phosphorus | about 12 mg / l |
| Magnesium | > 100 mg / l |
| Potassium | > 100 mg / l |
| Sodium | > 100 mg / l |

2. The formulation according to claim 1, wherein said colloidal solution also comprises at least one of iron, manganese, zinc.

3. The formulation according to one of claims 1 or 2, wherein said colloidal solution also comprises at least one of copper, selenium.

4. The formulation according to one of claims 1 to 3, wherein said colloidal solution comprises:
| | |
|---|---|
| Calcium | > 100mg / l |
| Chlorine | about 8 mg / l |
| Phosphorus | about 12 mg / l |
| Magnesium | > 100 mg / l |
| Potassium | > 100 mg / l |
| Sodium | > 100 mg / l |
| Total iron | > 250 mg / l |
| Manganese | about 9 mg / l |
| Zinc | > 50 mg / l |
| Copper | between 0 and 0.400 mg / l |
| Selenium | between 0 and 0.400 mg / l |

5. The formulation according to one of claims 1 to 3, wherein said colloidal solution comprises:
| | |
|---|---|
| Calcium | about 160 mg / l |
| Chlorine | about 8 mg / l |
| Phosphorus | about 12 mg / l |
| Magnesium | between 100 and 600 mg / l |
| Potassium | between 100 and 400 mg / l |
| Sodium | between 100 and 400 mg / l |
| Total iron | between 250 and 600 mg / l |
| Manganese | about 9 mg / l |
| Zinc | > 50 mg / l |
| Copper | between 0 and 0.400 mg / l |
| Selenium | between 0 and 0.400 mg / l |

6. The formulation according to one of claims 1 to 7, wherein said cyclodextrins are selected from alpha-cyclodextrin, beta-cyclodextrin, gamma-cyclodextrin, hydroxypropyl-beta-cyclodextrin, hydroxypropyl-gamma-cyclodextrin, other cyclodextrin derivatives and their derivatives or mixtures.

7. The formulation according to one of claims 1 to 3, wherein said colloidal solution is Plant Mineral Toddy (# 17601 Nutriva, Cabassi & Giuriati SpA IT).

8. Composition comprising the formulation according to one of claims 1 to 7, diluted in a powder selected from the group comprising maltodextrins, polydextrose, starches or mixtures thereof.

9. Food supplement comprising a formulation according to one of claims 1 to 7 or a composition according to claim 8.

10. Cosmetic comprising a formulation according to one of claims 1 to 7 or a composition according to claim 8.

11. A composition which includes:
a) the formulation according to one of claims 1 to 7;
b) one or more additional actives;
c) pharmaceutically acceptable excipients.

12. The composition according to claim 11 which is in the form of a powder, tablet, aqueous solution, oily solution, or capsule.

13. The composition according to one of claims 11 or 12 which is for oral administration.

14. The composition according to one of claims 11 or 12 which is for topical use.

15. The composition according to one of claims 11 to 13 which is in tablet form and comprises (% w / V): said formulation in an amount between 0.1 and 40%, one or more further active 0.1-40 %, bulking agents 1-50%, binders 5-40%, disintegrating agents 1-40%, sweeteners 0-20%, anti-aggregating agents 1-5%, lubricants 1-5%, filming agents 0-5%, dyes 0- 1%, aromas 0-1%.

16. The composition according to one of claims 11 to 14 which is in the form of a powder and comprises (% w/V): said formulation in an amount between 0.1 and 40%, one or more further active 0.1-40 %, bulking agents 1-50%, sweeteners 0-75%, antiplatelet agents 1-5%, buffering agents 5-10%, dyes 0-1%, flavours 0-10%.

17. The composition according to one of claims 11 to 14 which is an aqueous solution and comprises (% w / V): said formulation in an amount between 0.1 and 40%, one or more further active 0.1-10% , 1-95% water, 1-50% bulking agents, 0-60% sweeteners, 5-10% buffering agents, 0-1% dyes, 0-10% flavours, 0.05-0.2% preservatives, acidifiers 0.1-5%, thickeners 0.1-5%.

18. The composition according to one of claims 11 to 14 which is an oily solution and comprises (% w / V): said formulation in an amount between 0.1 and 40%, one or more further 0-10% active, oils 1-95%, 0-1% dyes, 0-10% flavours, 0.1-5% antioxidants, 0.1-5% thickeners.
